# EUROPEAN PATENT SPECIFICATION

(11) **EP 4 134 055 B1**
(45) Date of publication and mention of the grant of the patent: **29.05.2024**
(21) Application number: 22190202.6
(22) Date of filing: 12.08.2022
(51) Int. Cl.: A61G 12/00

(54) **MEDICAL STATION WITH MATERIAL COLLECTION POINTS AND CHANNELS**
MEDIZINISCHE STATION MIT MATERIALSAMMELPUNKTEN UND KANÄLEN
STATION MÉDICALE DOTÉE DE POINTS ET DE CANAUX DE COLLECTE DE MATÉRIAU

(30) Priority: 13.08.2021 US 202163232942 P
(43) Date of publication of application: 15.02.2023
(73) Proprietor: Carefusion 303 Inc., San Diego, California 92130 (US)
(72) Inventor: ARROYO, Noe, San Diego, 92130 (US); HOARD, David, San Diego, 92130 (US)
(74) Representative: Schulz, Oliver Frank Michael

(56) References cited:
- IT-A1- BO20 100 632
- US-A- 4 980 956
- US-A- 6 116 461

## Description

### TECHNICAL FIELD

The current subject matter described herein relates generally to a medical station. In particular, the current subject matter relates to a medical station with collection points and channels, receptacles, or any other structure for collection and diversion of materials.

### BACKGROUND

Medical stations may be incorporated in various clinical and healthcare environments. For example, a medical station may include a standing unit or base that incorporates a processing device such that a clinician or healthcare practitioner may operate and/or access the processing device at the standing unit or base. The processing device may be integral with or otherwise connected to the standing unit or base. The medical station may be incorporated with other stations and/or modules. From US 6,116,461 A an apparatus for the dispensing of drugs is known, wherein modular receptacles are filled and transported to automatic dispensing machines.

### SUMMARY

The invention is set out in the appended set of claims.

Aspects of the current subject matter relate to a medical station with collection points and channels to collect and divert materials away from sensitive areas of the medical station. In an example implementation, the medical station is a medical dispensing station or medication dispenser that is configured to dispense a medication.

In one aspect, there is disclosed a medical station, comprising: a base; and an upper module formed of a structure positioned atop the base, the upper module comprising a raised covering component and a planar support component, the raised covering component covering one or more electronic components. The raised covering component comprises a first top surface; a first recess formed within at least a portion of the first top surface; at least one channel extending from the first recess; and a plurality of sidewalls extending from respective edges of the first top surface, wherein the at least one channel extends over at least one of the plurality of sidewalls to divert spilled material away from the one or more electronic components. The planar support component comprises a second top surface extending from a distal end of a first sidewall of the plurality of sidewalls of the raised covering component, and a second recess formed within at least a portion of the second top surface.

The details of one or more variations of the subject matter described herein are set forth in the accompanying drawings and the description below. Other features and advantages of the subject matter described herein will be apparent from the description and drawings, and from the claims. The claims that follow this disclosure are intended to define the scope of the protected subject matter.

### DESCRIPTION OF THE DRAWINGS

The accompanying drawings, which are incorporated in and constitute a part of this specification, show certain aspects of the subject matter disclosed herein and, together with the description, help explain some of the principles associated with the disclosed implementations. In the drawings,
FIG. 1 is a perspective view of an upper module of a medical station consistent with implementations of the current subject matter;
FIG. 2 is a side view of an upper module of a medical station consistent with implementations of the current subject matter;
FIG. 3 is a top view of an upper module of a medical station consistent with implementations of the current subject matter;
FIG. 4 is a back view of an upper module of a medical station consistent with implementations of the current subject matter;
FIG. 5 depicts aspects of a medication station consistent with implementations of the current subject matter; and
FIG. 6 depicts aspects of a medication station consistent with implementations of the current subject matter.

When practical, similar reference numbers denote similar structures, features, or elements.

### DETAILED DESCRIPTION

Aspects of the current subject matter relate to a medical station with collection points and channels to collect and divert materials away from sensitive areas (or other areas) of the medical station. The medical station may be a standalone module or a component of a modular system. The medical station may be part of a medication management system that includes various modules and stations for the secure storage and dispensing of medication.

Aspects of the current subject matter provide a medical station that has a base and an upper module, where the upper module connects to or is otherwise integral with the base. The upper module is a structure or frame positioned atop the base. In some implementations, the upper module may connect to more than one type of base. In some implementations, the base is a standing unit or module. The base may be used to store medication, supplies, and/or documentation such as medical records or files. In some implementations, the base may be a medication dispensing unit. In some implementations, the base may include a refrigeration and/or freezer unit.

The medical station in accordance with implementations of the current subject matter is provided with features including collection points, channels, receptacles, and/or other structures to safely and securely contain and direct materials that may be spilled on the medical station. For example, as the medical station may include electronic components, such as one or more processing devices, and other valuable or sensitive components, there is a need to protect these components from accidental spills of liquids or other materials that may occur. The medical station includes dedicated areas to catch the materials, and in some instances divert the materials, in such a way that the electronic components and other components are not damaged.

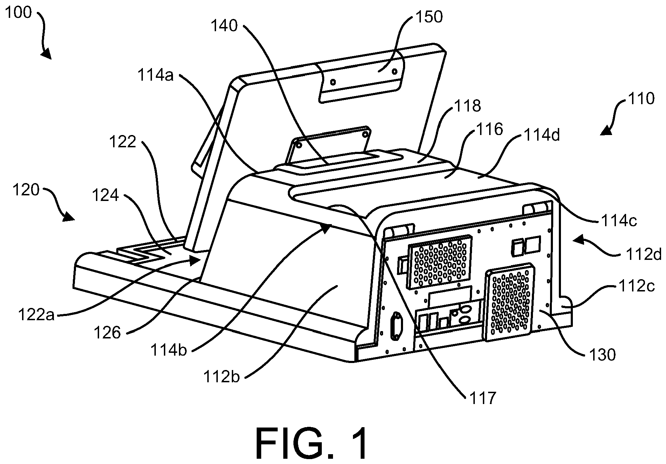

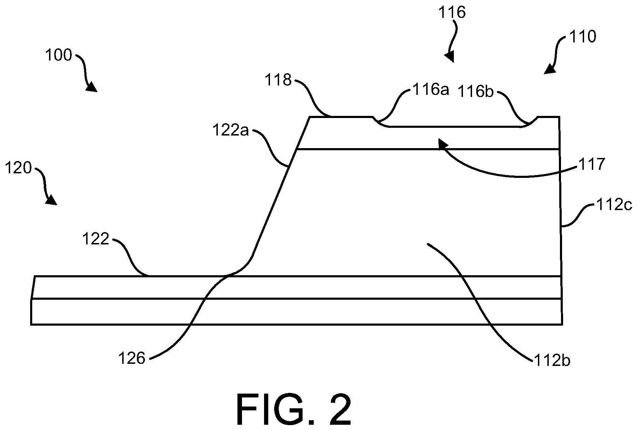

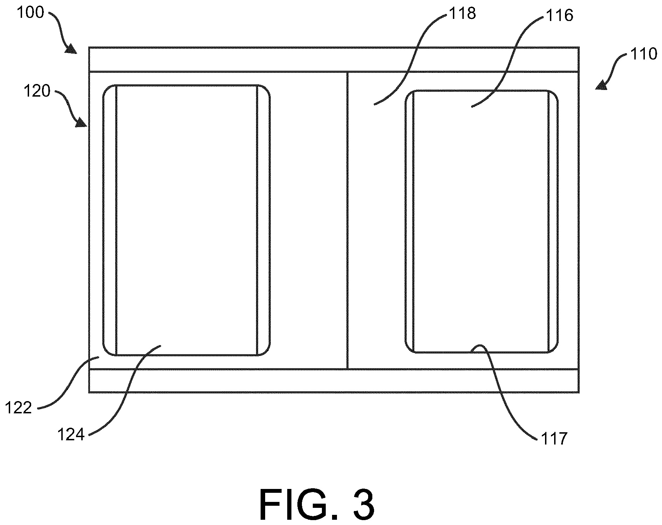

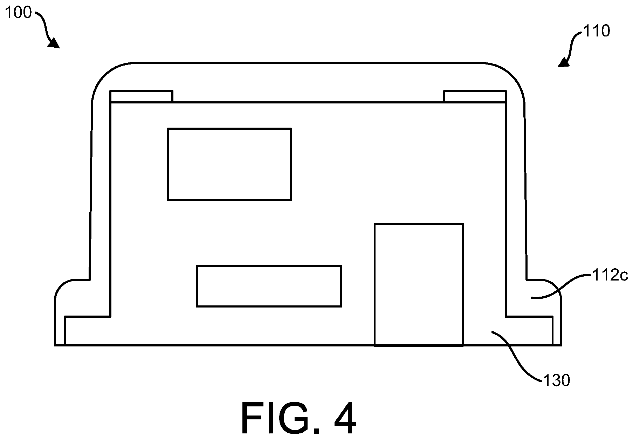

FIG. 1, FIG. 2, FIG. 3, and FIG. 4 depict various aspects of an upper module 100 of a medical station consistent with implementations of the current subject matter. FIG. 1 depicts a rear, perspective view of the upper module 100, FIG. 2 depicts a side view of the upper module 100, FIG. 3 depicts a top view of the upper module 100, and FIG. 4 depicts a rear view of the upper module 100.

The upper module 100 is shaped, configured and positioned on a base (not shown in FIG. 1 - FIG. 4) to cover and protect a processing device and to provide a work surface for healthcare workers. The upper module 100, according to aspects of the current subject matter, includes a raised covering component 110 and a planar support component 120. The raised covering component 110 is shaped and positioned to cover and contain within a processing device. The planar support component 120 is shaped and positioned in front of the raised covering component 110 to provide a work surface for healthcare workers. A display unit 150 may couple to the upper module 100 to allow for the healthcare workers to access and use the processing device. The planar support component 120 has a recessed work surface to hold or contain items (such as medications, pens, papers, or other items.) In an implementation, the recessed work surface is sized and shaped to contain or capture a volume of fluid (such as to contain 1 liter of liquid) should a spill occur.

With reference to FIG. 1 and FIG. 2, the raised covering component 110 has a first top surface 118 and a first recess 116 formed within at least a portion of the first top surface 118. A channel 117 extends from the first recess 116. The channel is configured to divert liquid in the first recess 116 away from sensitive electronic components in the front and back, such as to the left and right side of the first recess1 16. For example, the first recess 116 may have one or more edges or sides from which material contained in the first recess 116 may flow, allowing the material to be diverted from one or more sensitive areas of the medical station. In some implementations, more than one channel may extend from the first recess 116.

The raised covering component 110 has sidewalls 112a, 112b, 112c, and 112d extending from respective edges 114a, 114b, 114c, and 114d of the first top surface 118. The top surface 118 and the sidewalls 112a, 112b, 112c, and 112d form the protective structure (e.g., the raised covering component 110) in which the processing device of the medical station may be contained.

The planar support component 120 has a second top surface 122 that extends outward from a distal or bottom end 126 of the sidewall 112a of the raised covering component 110. A second recess 124 is formed within at least a portion of the second top surface 122. The second recess 124 is sized, shaped and configured to contain a liquid.

The first recess 116 may be defined by a first pair of opposing walls 116a and 116b, as shown in FIG. 2. The first pair of opposing walls 116a and 116b prevent spilled material from being directed to a front area of the raised covering component 110, where the display unit 150 may be positioned, and to a back area of the raised covering component 110, where access to the processing device may be provided. For example, on the sidewall 112c, a back plate 130 is provided and secured; see also FIGs. 1 and 4. The back plate 130 has various openings and connections to the processing device contained within the raised covering component 110. Thus, the opposing walls 116a and 116b prevent any spilled material from coming into contact with these sensitive areas. Moreover, the channel 117, which extends over the sidewall 112b from the first recess 116 between the opposing walls 116a and 116b, further diverts the spilled material from the display unit 150 and the back plate 130. In some implementations, the opposing walls 116a and 116b may be oriented and/or configured in a sloped orientation with respect to a bottom surface of the first recess 116. The slope can be oriented to direct fluid to any location such as to a rearward and/or frontward direction. Other structures can be included to direct fluid away from the station. For example, a lip can be included to direct fluid away from the front or rear surface so that fluid spills toward the floor away from the station.

Consistent with implementations of the current subject matter, two channels may be formed in the top surface 118 and extend from the first recess 116 and along the sidewalls 112b and 112d.

The second recess 124 of the planar support component 120 may be defined by a first pair of opposing walls and a second pair of opposing walls, the second pair of opposing walls oriented perpendicular to the first pair of opposing walls. One or more of the first pair of opposing walls and the second pair of opposing walls may be in a sloped orientation with respect to a bottom surface of the second recess 124. The second recess 124, formed in the second top surface 122, may serve to collect and contain spilled material in the front portion of the upper module 100.

According to some aspects of the current subject matter, the first recess 116 may have a concave configuration in which a middle portion or near middle portion of the first recess 116 is lower than surrounding portions. Similarly, the second recess 124 may have a concave configuration in which a middle portion or near middle portion of the second recess 124 is lower than surrounding portions.

In some implementations, the first top surface 118 and/or the second top surface 122 may have respective grated plates that fit within at least a portion of the respective recesses, the first recess 116 and the second recess 124. The grated plates may align with the first top surface 118 and/or the second top surface 122, and may have holes or openings through which a material that is spilled passes for collection and/or diversion.

As shown in FIG. 3, the first top surface 118 and the second top surface 122 are aligned in a side by side configuration, while at different heights from one another by virtue of the side walls 112a, 112b, 112c, and 112d that form the sides of the raised covering component 110. As seen in FIG. 3, and also in FIG. 1, the first top surface 118 includes a substantially flat area adjacent at least a portion of the first recess 116. The second top surface 122 includes a substantially flat area adjacent at least a portion of the second recess 124.

The first top surface 118, the first recess 116, the second top surface 122, and the second recess 124 may be formed from a hydrophobic material. The hydrophobic material may include a plastic or resin that repels liquid. In some implementations, portions of the surfaces or recesses may be formed from or coated with a hydrophobic material. Using hydrophobic material, either to form or coat a component, liquid can be diverted along a particular path and away from sensitive locations. Examples of hydrophobic materials include waxes, oils, alkyls, fluoroalkyls, prefluoroalkyls, or combinations or variants thereof. U.S. Patent No. 9,096,786 describes spill-proof or spill-resistant surfaces through the use of hydrophobic or oleophobic.

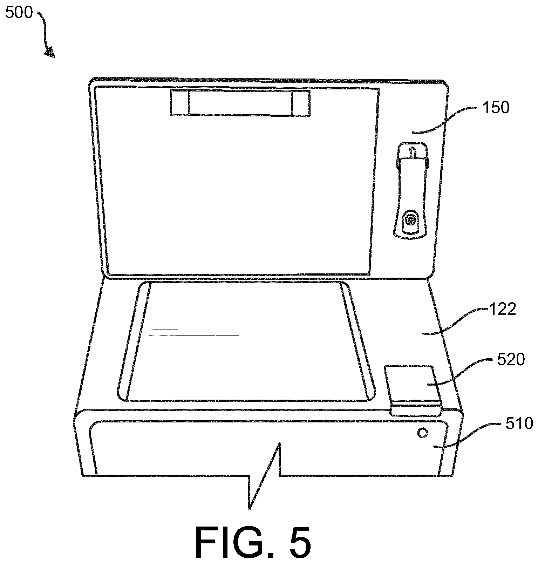

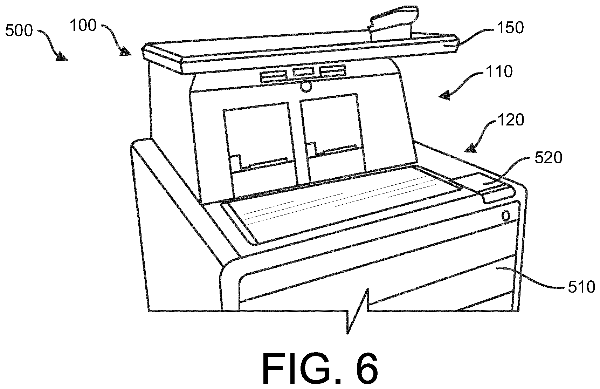

FIG. 5 and FIG. 6 depict aspects of a medical station 500 consistent with implementations of the current subject matter. The medical station 500 includes the upper module 100 having the raised covering component 110 and the planar support component 120 and a base 510. In some implementations, the base 510 and the upper module 100 are a single molded component. In some implementations, the upper module 100 is s releasably connectable to at least one portion of the base 510. The upper module 100 may form a hinged connection with at least one portion of the base 510. The first top surface 118 and the second top surface 122 of the upper module 100 are substantially perpendicular to base sidewalls of the base 510.

With reference still to FIG. 5 and FIG. 6, an electronic component 520 is positioned on or adjacent the second top surface 122 at a location accessible by a user. The electronic component 520 may be, for example, a card reader. Any portion of the second top surface 120 or the second recess 124 may be oriented to direct fluid to flow away from the electronic component 520 such as to avoid liquid damage to the electronic component.

Although the disclosure, including the figures, described herein may describe and/or exemplify these different variations separately, it should be understood that all or some, or components of them, may be combined.

Although various illustrative embodiments are described above, any of a number of changes may be made to various embodiments. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the claims.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. References to a structure or feature that is disposed "adjacent" another feature may have portions that overlap or underlie the adjacent feature.

Terminology used herein is for the purpose of describing particular embodiments only and is not intended to be limiting. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as, for example, "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings provided herein.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" "or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure.

Combinations of the above embodiments, and other embodiments not specifically described herein, are possible.

In the descriptions above and in the claims, phrases such as, for example, "at least one of" or "one or more of" may occur followed by a conjunctive list of elements or features. The term "and/or" may also occur in a list of two or more elements or features. Unless otherwise implicitly or explicitly contradicted by the context in which it used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." Use of the term "based on," above and in the claims is intended to mean, "based at least in part on," such that an unrecited feature or element is also permissible.

## Claims

1. A medical station, comprising:
a base; and
an upper module (100) formed of a structure positioned atop the base, the upper module (100) comprising a raised covering component (110) and a planar support component (120), the raised covering component (110) covering one or more electronic components,
the raised covering component (110) comprising:
a first top surface (118);
a first recess (116) formed within at least a portion of the first top surface (118);
at least one channel (117) extending from the first recess (116); and
a plurality of sidewalls (112a,...112d) extending from respective edges (114a,... 114d) of the first top surface (118), wherein the at least one channel (117) extends over at least one of the plurality of sidewalls (112a,...112d) to divert spilled material away from the one or more electronic components;
the planar support (120) component comprising:
a second top surface (122) extending from a distal end of a first sidewall (112a) of the plurality of sidewalls (112a,...112d) of the raised covering component (110); and
a second recess (124) formed within at least a portion of the second top surface (122).

2. The medical station of claim 1, wherein the first top surface (118) and the second top surface (122) are aligned in a side by side configuration at different heights from one another.

3. The medical station of claim 1, wherein the base and the upper module (100) comprise a single molded component.

4. The medical station of claim 1, wherein the upper module (100) is releasably connectable to at least one portion of the base.

5. The medical station of claim 1, wherein the upper module (100) forms a hinged connection with at least one portion of the base.

6. The medical station of claim 1, wherein the first top surface (118) and the second top surface (122) are substantially perpendicular to base sidewalls of the base.

7. The medical station of claim 1, wherein the first recess (116) is defined by at least a first pair of opposing walls (116a, 116b).

8. The medical station of claim 7, wherein the first pair of opposing walls (116a, 116b) are in a sloped orientation with respect to a bottom surface of the first recess (116).

9. The medical station of claim 7, wherein the at least one channel (117) extends between the first pair of two opposing walls (116a, 116b) and along one of the plurality of sidewalls (112a,...112d).

10. The medical station of claim 1, wherein the first top surface (118) comprises two channels, a first channel (117) extending from the first recess (116) and along a first of the plurality of sidewalls (112a,...112d), a second channel extending from the first recess (116) and along a second of the plurality of sidewalls (112a,...112d), the first of the plurality of sidewalls (112a,...112d) opposite the second of the plurality of sidewalls (112a,...112d).

11. The medical station of claim 1, wherein the second recess (124) is defined by a first pair of opposing walls and a second pair of opposing walls, the second pair of opposing walls oriented perpendicular to the first pair of opposing walls.

12. The medical station of claim 11, wherein one or more of the first pair of opposing walls and the second pair of opposing walls are in a sloped orientation with respect to a bottom surface of the second recess (124).

13. The medical station of claim 1, wherein the first recess (116) comprises a concave configuration in which a middle portion of the first recess (116) is lower than surrounding portions.

14. The medical station of claim 1, wherein the second recess (124) comprises a concave configuration in which a middle portion of the second recess (124) is lower than surrounding portions.

15. The medical station of claim 1, wherein the first top surface (118) further comprises a grated plate fitted within at least a portion of the first recess (116).

16. The medical station of claim 1, wherein the second top surface (122) further comprises a grated plate fitted within at least a portion of the second recess (124).

17. The medical station of claim 1, wherein the first top surface (118) comprises a substantially flat area adjacent at least a portion of the first recess (116).

18. The medical station of claim 1, wherein the second top surface (122) comprises a substantially flat area adjacent at least a portion of the second recess (124).

19. The medical station of claim 1, further comprising an attachment component affixed to the upper module, the attachment component configured to securely hold a display unit (150).

20. The medical station of claim 1, wherein the first recess (116) and/or the second recess (124) comprises a hydrophobic material.

21. The medical station of claim 1, wherein the medical station is part of a modular system.

22. The medical station of claim 1, wherein the medical station is a standalone module.

23. The medical station of claim 1, wherein the raised covering component (110) is configured to cover and protect a processing device.

24. The medical station of claim 23, further comprising a back plate (130) attached to one of the plurality of sidewalls (112c), the back plate (130) providing access to the processing device.

25. The medical station of claim 23, wherein the at least one channel (117) diverts a material contained in the first recess (116) from the back plate (130).

26. The medical station of claim 1, further comprising an electronic component on or adjacent the second top surface (122), wherein the second top surface (122) or second recess (124) is oriented to direct fluid to flow away from the electronic component such as to avoid liquid damage to the electronic component.

## Patentansprüche

1. Eine medizinische Station, umfassend:
einen Sockel; und
ein oberes Modul (100), das aus einer Struktur ausgebildet ist, die oben auf dem Sockel positioniert ist, wobei das obere Modul (100) eine erhöhte Abdeckungskomponente (110) und eine ebene Trägerkomponente (120) umfasst, wobei die erhöhte Abdeckungskomponente (110) eine oder mehrere elektronische Komponenten abdeckt,
wobei die erhöhte Abdeckungskomponente (110) umfasst:
eine erste obere Oberfläche (118);
eine erste Aussparung (116), die in mindestens einem Teil der ersten oberen Oberfläche (118) ausgebildet ist;
mindestens einen Kanal (117), der sich von der ersten Aussparung (116) aus erstreckt; und
eine Vielzahl von Seitenwänden (112a, ... 112d), die sich von jeweiligen Kanten (114a, ... 114d) der ersten oberen Oberfläche (118) erstrecken, wobei sich der mindestens eine Kanal (117) über mindestens eine der Vielzahl von Seitenwänden (112a, ... 112d) erstreckt, um verschüttetes Material von der einen oder mehreren elektronischen Komponenten wegzuleiten;
wobei die ebene Trägerkomponente (120) umfasst:
eine zweite obere Oberfläche (122), die sich von einem distalen Ende einer ersten Seitenwand (112a) der Vielzahl von Seitenwänden (112a, ... 112d) der erhöhten Abdecckomponente (110) erstreckt; und
eine zweite Aussparung (124), die in mindestens einem Teil der zweiten oberen Oberfläche (122) ausgebildet ist.

2. Medizinische Station nach Anspruch 1, wobei die erste obere Oberfläche (118) und die zweite obere Oberfläche (122) in einer nebeneinander liegenden Konfiguration in unterschiedlichen Höhen voneinander ausgerichtet sind.

3. Medizinische Station nach Anspruch 1, wobei der Sockel und das obere Modul (100) ein einziges Formteil umfassen.

4. Medizinische Station nach Anspruch 1, wobei das obere Modul (100) mit mindestens einem Teil des Sockels lösbar verbunden werden kann.

5. Medizinische Station nach Anspruch 1, wobei das obere Modul (100) mit mindestens einem Teil des Sockels eine gelenkige Verbindung ausbildet.

6. Medizinische Station nach Anspruch 1, wobei die erste obere Oberfläche (118) und die zweite obere Oberfläche (122) im Wesentlichen senkrecht zu Sockelseitenwänden des Sockels sind.

7. Medizinische Station nach Anspruch 1, wobei die erste Aussparung (116) durch mindestens ein erstes Paar gegenüberliegender Wände (116a, 116b) definiert ist.

8. Medizinische Station nach Anspruch 7, wobei das erste Paar gegenüberliegender Wände (116a, 116b) in einer schrägen Ausrichtung in Bezug auf eine Bodenfläche der ersten Aussparung (116) sind.

9. Medizinische Station nach Anspruch 7, wobei sich der mindestens eine Kanal (117) zwischen dem ersten Paar von zwei gegenüberliegenden Wänden (116a, 116b) und entlang einer der Vielzahl von Seitenwänden (112a, ... 112d) erstreckt.

10. Medizinische Station nach Anspruch 1, wobei die erste obere Oberfläche (118) zwei Kanäle umfasst, einen ersten Kanal (117), der sich von der ersten Aussparung (116) und entlang einer ersten der Vielzahl von Seitenwänden (112a, ... 112d) erstreckt, einen zweiten Kanal, der sich von der ersten Aussparung (116) und entlang einer zweiten der Vielzahl von Seitenwänden (112a, ... 112d) erstreckt, wobei die erste der Vielzahl von Seitenwänden (112a, ... 112d) gegenüber der zweiten der Vielzahl von Seitenwänden (112a, ... 112d).

11. Medizinische Station nach Anspruch 1, wobei die zweite Aussparung (124) durch ein erstes Paar gegenüberliegender Wände und einem zweiten Paar gegenüberliegender Wände definiert ist, wobei das zweite Paar gegenüberliegender Wände senkrecht zu dem ersten Paar gegenüberliegender Wände ausgerichtet.

12. Medizinische Station nach Anspruch 11, wobei eine oder mehrere des ersten Paars gegenüberliegender Wände und des zweiten Paars gegenüberliegender Wände in einer schrägen Ausrichtung in Bezug auf eine Bodenfläche der zweiten Aussparung (124) sind.

13. Medizinische Station nach Anspruch 1, wobei die erste Aussparung (116) eine konkave Konfiguration umfasst, in welcher ein mittlerer Teil der ersten Aussparung (116) niedriger ist als umgebende Teile.

14. Medizinische Station nach Anspruch 1, wobei die zweite Aussparung (124) eine konkave Konfiguration umfasst, in welcher ein mittlerer Teil der zweiten Aussparung (124) niedriger ist als umgebende Teile.

15. Medizinische Station nach Anspruch 1, wobei die erste obere Oberfläche (118) weiter eine Rostplatte umfasst, die in mindestens einem Teil der ersten Ausnehmung (116) eingepasst ist.

16. Medizinische Station nach Anspruch 1, wobei die zweite obere Oberfläche (122) weiter eine Rostplatte umfasst, die in mindestens einem Teil der zweiten Aussparung (124) eingepasst ist.

17. Medizinische Station nach Anspruch 1, wobei die erste obere Oberfläche (118) einen im Wesentlichen flachen Bereich umfasst, der an mindestens einem Teil der ersten Aussparung (116) angrenzt.

18. Medizinische Station nach Anspruch 1, wobei die zweite obere Oberfläche (122) einen im Wesentlichen flachen Bereich umfasst, der an mindestens einem Teil der zweiten Aussparung (124) angrenzt.

19. Medizinische Station nach Anspruch 1, weiter umfassend eine am oberen Modul befestigte Befestigungskomponente, wobei die Befestigungskomponente so konfiguriert ist, dass sie eine Anzeigeeinheit (150) sicher hält.

20. Medizinische Station nach Anspruch 1, wobei die erste Aussparung (116) und/oder die zweite Aussparung (124) ein hydrophobes Material umfasst.

21. Die medizinische Station nach Anspruch 1, wobei die medizinische Station Teil eines modularen Systems ist.

22. Die medizinische Station nach Anspruch 1, wobei die medizinische Station ein eigenständiges Modul ist.

23. Medizinische Station nach Anspruch 1, wobei die erhöhte Abdeckkomponente (110) konfiguriert ist, ein Bearbeitungsgerät abzudecken und zu schützen.

24. Medizinische Station nach Anspruch 23, weiter umfassend eine Rückplatte (130), die an einer der Vielzahl von Seitenwänden (112c) angebracht ist, wobei die Rückplatte (130) einen Zugang zu dem Bearbeitungsgerät bietet.

25. Medizinische Station nach Anspruch 23, wobei der mindestens eine Kanal (117) ein in der ersten Aussparung (116) enthaltenes Material von der Rückplatte (130) ableitet.

26. Medizinische Station nach Anspruch 1, weiter umfassend eine elektronische Komponente auf oder angrenzend an der zweiten oberen Oberfläche (122), wobei die zweite obere Oberfläche (122) oder zweite Aussparung (124) so ausgerichtet ist, um eine Flüssigkeit von der elektronischen Komponente wegzuleiten, um Flüssigkeitsschäden an der elektronischen Komponente zu vermeiden.

## Revendications

1. Une station médicale comprenant
une base ; et
un module supérieur (100) formé d'une structure positionnée sur la base, le module supérieur (100) comprenant un composant de couverture surélevé (110) et un composant de support planaire (120), le composant de couverture surélevé (110) couvrant un ou plusieurs composants électroniques,
le composant de couverture surélevé (110) comprenant :
une première surface supérieure (118) ;
un premier renfoncement (116) formé à l'intérieur d'au moins une partie de la première surface supérieure (118) ;
au moins un canal (117) s'étendant à partir du premier renfoncement (116) ; et
une pluralité de parois latérales (112a,...112d) s'étendant à partir des bords respectifs (114a,...114d) de la première surface supérieure (118), dans laquelle au moins un canal (117) s'étend sur au moins une de la pluralité de parois latérales (112a,...112d) pour dévier de matériau renversé loin d'un ou plusieurs composants électroniques ;
le composant de support planaire (120) comprenant :
une deuxième surface supérieure (122) s'étendant à partir d'une extrémité distale d'une première paroi latérale (112a) de la pluralité de parois latérales (112a,...112d) du composant de couverture surélevé (110) ; et
un second renfoncement (124) formé à l'intérieur d'au moins une partie de la deuxième surface supérieure (122).

2. Station médicale de la revendication 1, dans laquelle la première surface supérieure (118) et la seconde surface supérieure (122) sont alignées dans une configuration côte à côte à des hauteurs différentes l'une de l'autre.

3. Station médicale de la revendication 1, dans laquelle la base et le module supérieur (100) comprennent un seul composant moulé.

4. Station médicale de la revendication 1, dans laquelle le module supérieur (100) peut être relié de manière amovible à au moins une partie de la base.

5. Station médicale de la revendication 1, dans laquelle le module supérieur (100) forme une liaison articulée avec au moins une partie de la base.

6. Station médicale de la revendication 1, dans laquelle la première surface supérieure (118) et la deuxième surface supérieure (122) sont sensiblement perpendiculaires aux parois latérales de la base.

7. Station médicale de la revendication 1, dans laquelle le premier renfoncement (116) est défini par au moins une première paire de parois opposées (116a, 116b).

8. Station médicale de la revendication 7, dans laquelle la première paire de parois opposées (116a, 116b) sont inclinées par rapport à la surface inférieure du premier renfoncement (116).

9. Station médicale de la revendication 7, dans laquelle au moins un canal (117) s'étend entre la première paire de deux parois opposées (116a, 116b) et le long d'une de la pluralité de parois latérales (112a,...112d).

10. Station médicale de la revendication 1, dans laquelle la première surface supérieure (118) comprend deux canaux, un premier canal (117) s'étendant à partir du premier renfoncement (116) et le long d'un premier de la pluralité de parois latérales (112a,...112d), un second canal s'étendant à partir du premier renfoncement (116) et le long d'un second de la pluralité de parois latérales (112a,...112d), le premier de la pluralité de parois latérales (112a,...112d) opposé au second de la pluralité de parois latérales (112a,...112d).

11. Station médicale de la revendication 1, dans laquelle le second renfoncement (124) est défini par une première paire de parois opposées et une deuxième paire de parois opposées, la deuxième paire de parois opposées orientée perpendiculairement à la première paire de parois opposées.

12. Station médicale de la revendication 11, dans laquelle une ou plusieurs des premières paires de parois opposées et des deuxièmes paires de parois opposées sont inclinées par rapport à la surface inférieure du second renfoncement (124).

13. Station médicale de la revendication 1, dans laquelle le premier renfoncement (116) comprend une configuration concave dans laquelle une partie centrale du premier renfoncement (116) est plus basse que des parties environnantes.

14. Station médicale de la revendication 1, dans laquelle le second renfoncement (124) comprend une configuration concave dans laquelle une partie centrale du second renfoncement (124) est plus basse que des parties environnantes.

15. Station médicale de la revendication 1, dans laquelle la première surface supérieure (118) comprend en outre une plaque grillagée insérée dans au moins une partie du premier renfoncement (116).

16. Station médicale de la revendication 1, dans laquelle la seconde surface supérieure (122) comprend en outre une plaque grillagée insérée dans au moins une partie du second renfoncement (124).

17. Station médicale de la revendication 1, dans laquelle la première surface supérieure (118) comprend une zone sensiblement plate adjacente à au moins une partie du premier renfoncement (116).

18. Station médicale de la revendication 1, dans laquelle la deuxième surface supérieure (122) comprend une zone sensiblement plate adjacente à au moins une partie du second renfoncement (124).

19. Station médicale de la revendication 1, comprenant en outre un composant de fixation fixé au module supérieur, le composant de fixation étant configuré pour maintenir en toute sécurité une unité d'affichage (150).

20. Station médicale de la revendication 1, dans laquelle le premier renfoncement (116) et/ou le second renfoncement (124) comprend un matériau hydrophobe.

21. Station médicale de la revendication 1, dans laquelle la station médicale fait partie d'un système modulaire.

22. Station médicale de la revendication 1, dans laquelle la station médicale est un module autonome.

23. Station médicale de la revendication 1, dans laquelle le composant de couverture surélevé (110) est configuré pour couvrir et protéger un dispositif de traitement.

24. Station médicale de la revendication 23, comprenant en outre une plaque arrière (130) fixée à une de la pluralité de parois latérales (112c), la plaque arrière (130) permettant d'accéder au dispositif de traitement

25. Station médicale de la revendication 23, dans laquelle au moins un canal (117) dévie un matériau contenu dans le premier renfoncement (116) de la plaque arrière (130).

26. Station médicale de la revendication 1, comprenant en outre un composant électronique sur ou adjacent à la seconde surface supérieure (122), dans laquelle la seconde surface supérieure (122) ou le second renfoncement (124) est orienté de manière à diriger un fluide à l'écart du composant électronique afin d'éviter que le liquide n'endommage le composant électronique.
